(19) Europäisches Patentamt
European Patent Office
Office européen des brevets

(11) Publication number: **0 486 749 A1**

(12) **EUROPEAN PATENT APPLICATION**

(21) Application number: **91105774.3**

(51) Int. Cl.5: **B41M 5/136**, C07C 229/62

(22) Date of filing: **11.04.91**

(30) Priority: **22.11.90 JP 316118/90**

(43) Date of publication of application:
**27.05.92 Bulletin 92/22**

(84) Designated Contracting States:
**DE FR GB**

(71) Applicant: **HODOGAYA CHEMICAL CO., LTD.**
**4-2, Toranomon 1-chome Minato-ku**
**Tokyo(JP)**

(72) Inventor: **Wakasugi, Kazuyuki, Hodogaya Chem.Co.Ltd.**
**Central Research Lab., 2-30, Oji 6-chome**
**Kita-ku, Tokyo(JP)**
Inventor: **Kikkawa, Katsumasa, Hodogaya Chem.Co.Ltd.**
**Central Research Lab., 2-30, Oji 6-chome**
**Kita-ku, Tokyo(JP)**
Inventor: **Kaneko, Kazuo, Hodogaya Chem.Co.Ltd.**
**Central Research Lab., 2-30, Oji 6-chome**
**Kita-ku, Tokyo(JP)**
Inventor: **Yamaguchi, Masahiko, Hodogaya Chem.Co.Ltd.**
**Central Research Lab., 2-30, Oji 6-chome**
**Kita-ku, Tokyo(JP)**

(74) Representative: **Wächtershäuser, Günter, Dr.**
**Tal 29**
**W-8000 München 2(DE)**

(54) **Triarylmethane compounds and pressure sensitive recording material.**

(57) A triarylmethane compound of the following formula (I):

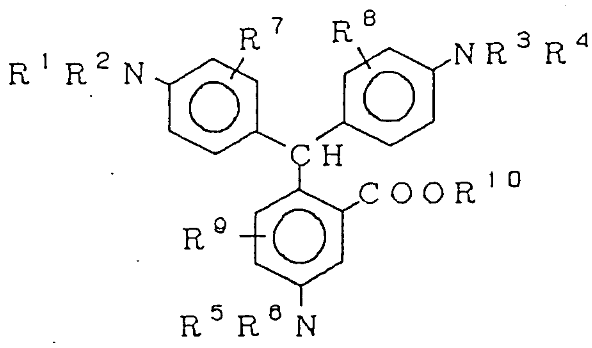

(I)

wherein each of $R^1$, $R^2$, $R^3$, $R^4$, $R^5$ and $R^6$ independently represents a $C_{1-8}$ alkyl group, a cycloalkyl group, an alkoxyalkyl group, or a benzyl or phenyl group which may be substituted by a halogen atom, an alkyl group or an alkoxy group, each of $R^7$, $R^8$ and $R^9$ independentlyrepresents a hydrogen atom, a halogen atom, an alkyl group, an alkoxy group, or an alkoxyalkyl group, and $R^{10}$ represents a $C_{1-8}$ alkyl group, an alkoxyalkyl group, a cycloalkyl group, or a benzyl or phenyl group which may be substituted by a halogen atom, an alkyl group, an alkoxy group or an alkoxyalkyl group.

EP 0 486 749 A1

The present invention relates to novel triarylmethane compounds and a pressure sensitive recording material comprising at least one of such compounds.

Color formers which are colorless or slightly colored basic dyes, have been widely used as pressure sensitive recording materials.

Typical examples of color formers commonly employed in such recording materials include, for example, crystal violet lactone, benzoylleucomethylene blue, 2-dibenzylamino-6-diethylaminofluorane, 1,3-dimethyl-6-diethylaminofluorane, 3-(4,4'-bis(N-methylanilino)benzohydryl)-9-butylcarbazole and 2-anilino-3-methyl-6-diethylaminofluorane.

Further, various color formers have been effectively used in combination to realize various performances such as the desired color, color density, color forming speed and storage stability depending upon the particular application of such recording materials. For example, in a blue color forming pressure sensitive recording sheet, crystal violet lactone and benzoylleucomethylene blue have been used in combination as the main components.

Crystal violet lactone is a color former which is capable of forming a clear blue color very quickly, but it has a drawback that the formed image tends to easily fade out due to poor light resistance. To complement the drawback, it has been proposed to mix benzoylleucomethylene blue which is a color former of slow color forming type in an attempt to overcome the color fading of a record image. However, this benzoylleucomethylene blue has a drawback that it tends to lead to coloring of a microcapsule slurry-coated sheet (hereinafter referred to as "CB sheet"), when the sheet is exposed to light.

It is an object of the present invention to overcome the drawbacks of such conventional color formers and to present a novel compound which is useful as a color former excellent in light resistance and to provide a recording material having the above drawbacks of conventional pressure sensitive recording materials substantially overcome.

Thus, the present invention provides a triarylmethane compound of the following formula (I):

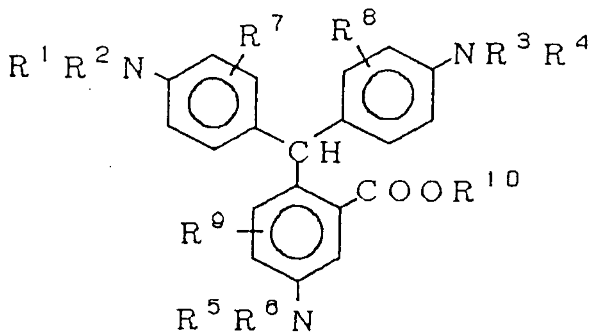

(I)

wherein each of $R^1$, $R^2$, $R^3$, $R^4$, $R^5$ and $R^6$ independently represents a $C_{1-8}$ alkyl group, a cycloalkyl group, an alkoxyalkyl group, or a benzyl or phenyl group which may be substituted by a halogen atom, an alkyl group or an alkoxy group, each of $R^7$, $R^8$ and $R^9$ independentlyrepresents a hydrogen atom, a halogen atom, an alkyl group, an alkoxy group, or an alkoxyalkyl group, and $R^{10}$ represents a $C_{1-8}$ alkyl group, an alkoxyalkyl group, a cycloalkyl group, or a benzyl or phenyl group which may be substituted by a halogen atom, an alkyl group, an alkoxy group or an alkoxyalkyl group.

Further, the present invention provides a pressure sensitive recording material comprising at least one member selected from the group consisting of triarylmethane compounds of the above formula (I).

The triarylmethane compound of the formula (I) is a color former capable of gradually forming a clear blue color when brought in contact with a developer such as an inorganic acid, an organic acid, a phenyl compound or a derivative or metal salt thereof, or an electron accepting substance such as an oxidizing agent, for example, acid clay, 3,5-di-t-butylsalicylic acid, bis(3-chlorophenyl)thiourea, 4,4'-isopropylidenediphenyl, zinc-4-nitrobenzoate or benzyl 4-hydroxybenzoate. Further, a pressure sensitive recording sheet prepared by using this color former has an excellent characteristic that the microcapsule slurry-coated sheet will undergo no substantial coloring upon exposure.

Thus, the present invention provides a recording material having the drawbacks of the conventional pressure sensitive recording materials substantially overcome.

In the present invention, typical examples of the developer used to obtain a pressure sensitive recording material, include bentonite, zeolite, acid clay, activated clay, silica gel, zinc oxide, zinc chloride, aluminum chloride, zinc bromide, a salicylic acid derivative such as 3-t-butylsalicylic acid, zinc 3,5-di-t-butylsalicylate or salicylanilide, a thiourea derivative such as bis(3-chlorophenyl)thiourea, a phenyl derivative such as 4-t-butylphenol, a novolak type phenol resin, a benzophenone derivative such as 4-hydroxybenzophenone, a benzoic acid derivative such as 4-nitrobenzoic acid, zinc 4-nitrobenzoate or ethyl 4-hydroxybenzoate, a naphthoic acid derivative such as 1-hydroxy-2-naphthoic acid or zinc 2-hydroxy-6-naphthoate, a phenylsulfone derivative such as 4-hydroxydiphenylsulfone, and an organic acid such as maleic acid, succinic acid, malic acid or stearic acid.

In the present invention, these color formers may be used alone or in combination as a mixture of two or more for the preparation of a pressure sensitive recording material.

In the present invention, typical examples of the solvent for the color former to be used to obtain the pressure sensitive recording material, include Hyzol SAS-296 (dialkylnaphthalene b.p. = 302 °C-309 °C; product of Nippon Petro Chemicals Co., Ltd.) and KMC-113 (diarylalkane b.p. = 290 °C-296 °C; product of Kureha Chemical Industries Co., Ltd.). However, solvents of monoisopropyldiphenyl type, alkylbenzene type, alkylbiphenyl type, alkylnaphthalene type, diarylethane type, hydrogenated terphenyl type and chlorinated paraffin type may also be used alone or in combination as a mixture.

Further, in the present invention, a coacervation method, an interfacial polymerization method or an in-situ method may be used as the capsulation method to obtain a pressure sensitive recording material.

The triarylmethane compound of the formula (I) can readily be prepared by the following common method:

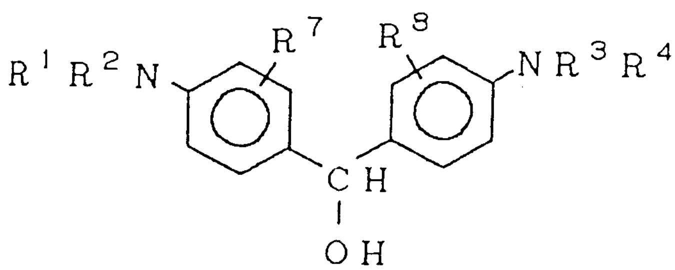

(II)

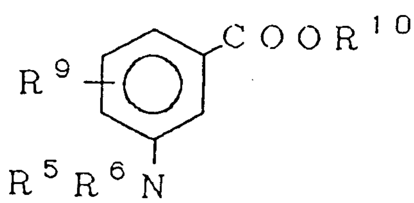

(III)

Namely, a 4,4'-diaminobenzhydrol derivative of the above formula (II) and a 3-aminobenzoic acid ester derivative of the above formula (III) are subjected to dehydration condensation in water or in an aqueous organic solvent e.g. an alcohol such as methyl alcohol or ethyl alcohol, a glycol such as ethylene glycol, tetrahydrofran or acetonitrile in the presence of an acid such as hydrochloric acid, hydrobromic acid, sulfuric acid, p-toluene sulufuric acid, formic acid or acetic acid, to readily obtain the triarylmethane compound of the formula (I). In the above formulas (II) and (III), $R^1$ to $R^{10}$ are as defined with respect to the formula (I).

Specific examples of the triarylmethane compounds thus obtained are shown in Table 1. The color in the Table is the color formed on silica gel.

Table 1

| $R^1$ | $R^2$ | $R^3$ | $R^4$ | $R^5$ | $R^6$ | $R^7$ | $R^8$ | $R^9$ | $R^{10}$ | Color |
|---|---|---|---|---|---|---|---|---|---|---|
| $CH_3$ | $CH_3$ | $CH_3$ | $CH_3$ | $CH_3$ | $CH_3$ | H | H | H | $CH_3$ | blue |
| $CH_3$ | $CH_3$ | $CH_3$ | $CH_3$ | $CH_3$ | $CH_3$ | H | H | H | $C_2H_5$ | blue |
| $CH_3$ | $CH_3$ | $CH_3$ | $CH_3$ | $CH_3$ | $CH_3$ | H | H | H | $C_3H_7$ | blue |
| $CH_3$ | $CH_3$ | $CH_3$ | $CH_3$ | $CH_3$ | $CH_3$ | H | H | H | $C_4H_9$ | blue |
| $CH_3$ | $CH_3$ | $CH_3$ | $CH_3$ | $CH_3$ | $CH_3$ | H | H | H | $C_6H_{13}$ | blue |
| $CH_3$ | $CH_3$ | $CH_3$ | $CH_3$ | $CH_3$ | $CH_3$ | H | H | H | $C_8H_{17}$ | blue |
| $CH_3$ | $CH_3$ | $CH_3$ | $CH_3$ | $CH_3$ | $CH_3$ | H | H | H | $C_2H_4OC_2H_5$ | blue |
| $CH_3$ | $CH_3$ | $CH_3$ | $CH_3$ | $CH_3$ | $CH_3$ | 2-$CH_3$ | 2-$CH_3$ | H | $CH_3$ | blue |
| $CH_3$ | $CH_3$ | $CH_3$ | $CH_3$ | $CH_3$ | $CH_3$ | 2-$CH_3$ | 2-$CH_3$ | H | $C_3H_7$ | blue |
| $CH_3$ | $CH_3$ | $CH_3$ | $CH_3$ | $CH_3$ | $CH_3$ | 2-$CH_3$ | 2-$CH_3$ | H | $C_4H_9$ | blue |
| $CH_3$ | $CH_3$ | $CH_3$ | $CH_3$ | $CH_3$ | $CH_3$ | 2-Cl | 2-Cl | H | $CH_3$ | blue |
| $CH_3$ | $CH_3$ | $CH_3$ | $CH_3$ | $CH_3$ | $CH_3$ | H | H | 5-$CH_3$ | $CH_3$ | blue |
| $C_2H_5$ | $C_2H_5$ | $C_2H_5$ | $C_2H_5$ | $CH_3$ | $CH_3$ | H | H | H | $CH_3$ | blue |
| $C_2H_5$ | $C_2H_5$ | $C_2H_5$ | $C_2H_5$ | $CH_3$ | $CH_3$ | 2-$OC_2H_5$ | 2-$OC_2H_5$ | H | $CH_3$ | blue |
| $CH_3$ | 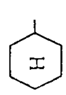 | $CH_3$ | 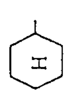 | H | H | H | H | H | $CH_3$ | blue |
| $CH_3$ | 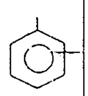 | $CH_3$ | 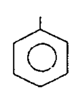 | H | H | H | H | H | $CH_3$ | blue |
| $CH_3$ | 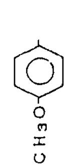 | $CH_3$ | 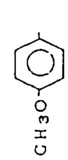 | H | H | H | H | H | $CH_3$ | blue |
| $CH_3$ | 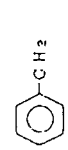 | $CH_3$ | 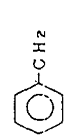 | H | H | H | H | H | $CH_3$ | blue |
| $CH_3$ | 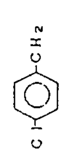 | $CH_3$ | 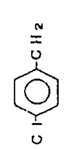 | H | H | H | H | H | $CH_3$ | blue |
| $CH_3$ | $CH_3$ | $CH_3$ | $CH_3$ | $CH_3$ | $CH_3$ | H | H | H | 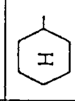 | blue |
| $CH_3$ | $CH_3$ | $CH_3$ | $CH_3$ | $CH_3$ | $CH_3$ | H | H | H | 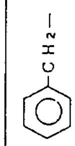 | blue |
| $CH_3$ | $CH_3$ | $CH_3$ | $CH_3$ | $CH_3$ | $CH_3$ | H | H | H | 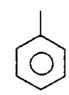 | blue |

Now, the process for producing the triarylmethane compounds of the present invention will be described in detail with reference to Preparation Examples. In the following description, "parts" means "parts by weight", and "%" means "% by weight".

Preparation Example 1

To a dilute hydrochloric acid aqueous solution prepared by diluting 25 parts of 35% hydrochloric acid

with 130 parts of water, 30 parts of 4,4'-bisdimethylaminobenzhydrol and 22 parts of methyl 3-dimethylaminobenzoate were dissolved and reacted at a temperature of from 50 to 60° C for 5 hours. Then, 10 parts of active carbon was added thereto, and the mixture was thoroughly mixed. After removing active carbon by filtration, 150 parts of 10% sodium hydroxide and 100 parts of toluene were added to the filtrate and thoroughly mixed. The toluene layer was separated, then washed with 100 parts of water and separated. Then, this toluene layer was thoroughly dried over anhydrous sodium salfate. Then, this toluene layer was concentrated, and 100 parts of methanol was added to the residue thereby obtained. Precipitated crystals were collected by filtration and recrystallized from a solvent mixture comprising 50 parts of methanol and 50 parts of acetone to obtain 35 parts of bis((4-dimethylamino)phenyl)-(2-methoxycarbonyl-4-dimethylaminophenyl)methane. This product had a melting point of from 155.6 to 155.8° C, and it gradually formed a clear blue color on silica gel TLC and showed excellent light resistance.

Preparation Example 2

The treatment was conducted in the same manner as in Preparation Example 1 except that 25 parts of isopropyl 3-dimethylaminobenzoate was used instead of 22 parts of methyl 3-dimethylaminobenzoate, to obtain 36 parts of bis((4-dimethylamino)phenyl)-(2-isopropoxycarbonyl-4-dimethylaminophenyl)methane. This product had a melting point of from 120.0 to 122.2° C, and it gradually formed a clear blue color on silica gel TLC and showed excellent light resistance.

Preparation Example 3

The treatment was conducted in the same manner as in Preparation Example 1 except that 28 parts of butyl 3-dimethylaminobenzoate was used instead of 22 parts of methyl 3-dimethylaminobenzoate, to obtain 33 parts of bis((4-dimethylamino)phenyl)-(2-n-butoxycarbonyl-4-dimethylaminophenyl)methane. This product had a melting point of from 78.2 to 78.7° C, and it gradually formed a clear blue color on silica gel TLC and showed excellent light resistance.

Preparation Example 4

The treatment was conducted in the same manner as in Preparation Example 1 except that 3 parts of bis(2-methyl-4-dimethylaminophenyl)methanol and 3 parts of methyl 3-dimethylaminobenzoate were used instead of 30 parts of 4,4'-dimethylaminobenzhydrol and 22 parts of methyl 3-dimethylaminobenzoate, to obtain bis((2-methyl-4-dimethylamino)phenyl)-(2-methoxycarbonyl-4-dimethylaminophenyl)methane. This compound gradually formed a clear blue color on silica gel TLC and showed excellent light resistance.

Preparation Example 5

The treatment was conducted in the same manner as in Preparation Example 4 except that 3 parts of ethyl 3-dimethylaminobenzoate was used instead of 3 parts of methyl 3-dimethylaminobenzoate, to obtain bis((2-methyl-4-dimethylamino)phenyl)-(2-ethoxycarbonyl-4-dimethylaminophenyl)methane. This compound gradually formed a clear blue color on silica gel TLC and showed excellent light resistance.

Now, data for comparison with Examples of the present invention were prepared. In the following, "parts" means "parts by weight", and "%" means "% by weight".

Comparative Example 1

To examine the solubility of a color former in oil, the solubility of benzoylleucomethylene blue in KMC-113 (product of Kureha Chemical Industries Co., Ltd.) as a typical solvent commonly employed, was investigated.

As a result, it showed a solubility of 2.5% at 50° C.

Comparative Example 2

3% of benzoylleucomethylene blue was dissolved in KMC-113 to obtain a solution. Then, 45 parts of this solution was added to 67.5 parts of an aqueous solution of 5% Scripset #520 (styrene maleic anhydride copolymer, manufactured by Monsant Company) adjusted to pH 4.0, and the mixture was emulsified by a homomixer under a condition of 6,500 rpm. To this emulsion, melamine prepolymer obtained by adjusting a

mixture comprising 5.6 parts of melamine, 37.4 parts of water and 17 parts of 37% formaline to pH 9, followed by a methylol-conversion reaction at 60 ° C for 30 minutes, was added. The liquid temperature was adjusted to 80 ° C, and a methylene-conversion reaction was conducted for 60 minutes under stirring to obtain a microcapsule slurry of a benzoylleucomethylene blue-dissolved oil.

This microcapsule slurry was coated on one side of an ordinary paper by means of a wire bar coater to form a layer of uniform thickness, followed by drying to obtain a CB sheet. The initial color density of the CB sheet thus obtained, was measured by means of Macbeth reflection color density tester RD-918 and found to be 0.05.

Further, this CB sheet was exposed to fade meter light for 10 minutes, or exposed at 40 ° C under relative humidity of 90% for 24 hours, or exposed at 100 ° C for 10 hours. Then, the color densities of the respective CB sheets were measured and found to be 0.24, 0.06 and 0.05, respectively.

Further, a fresh CB sheet to examine the color forming performance and a CB sheet exposed to fade mater light for 10 minutes to examine the light resistance color forming performance, were respectively put on clay type developer sheets as typical commercially available pressure sensitive developer sheets, followed by pressing under a pressure of 500 kg/cm$^2$ at 28 mmØ by a hydraulic pressing machine, whereby developed blue color record images were obtained respectively.

The color densities of the record images thus obtained were measured and found to be 0.85 and 0.76, respectively.

Further, to examine the light resistance against color fading, a record image obtained by a fresh CB sheet was exposed to fade meter light for one hour, and the reflection density of the record image was measured and found to be 0.82.

In the same manner as the above test, a resin type developer sheet and a metal salt of a salicylic acid derivative type developer sheet as typical commercially available pressure sensitive developer sheets, were tested for the light resistance against color fading.

Now, the present invention will be described in further detail. In the following, "parts" means "parts by weight", and "%" means "% by weight".

Example 1

To examine the solubility in oil of the triarylmethane compounds of the present invention, the solubility in KMC-113 (product of Kureha Chemical Industries Co., Ltd.) as a typical solvent was examined.

As a result, each of bis((4-dimethylamino)phenyl)-(2-methoxycarbonyl-4-dimethylaminophenyl)methane, bis((4-dimethylamino)phenyl)-(2-isopropoxycarbonyl-4-dimethylaminophenyl)methane and bis((4-dimethylamino)phenyl)-(2-n-butylcarbonyl-4-dimethylaminophenyl)methane showed a solubility of at least 10% at 50 ° C.

Example 2

45 parts of a solution prepared by dissolving 3% of bis((4-dimethylamino)phenyl)-(2-methoxycarbonyl-4-dimethylaminophenyl)methane in KMC-113 (product of Kureha Chemical Industries Co., Ltd.) was added to 67.5 parts of an aqueous solution of 5% Scripset #520 (styrene-maleic anhydride copolymer, manufactured by Monsant Company) adjusted to pH 4.0, and the mixture was emulsified by a homomixer under a condition of 6,500 rpm. To this emulsion, melamine prepolymer obtained by adjusting a mixture comprising 5.6 parts of melamine, 37.4 parts of water and 17 parts of 37% formaline to pH 9, followed by methylol-convertion reaction at 60 ° C for 30 minutes, was added. The liquid temperature was adjusted to 80 ° C, and methylene-conversion reaction was conducted under stirring for 60 minutes to obtain a microcapsule slurry of a bis((4-dimethylamino)phenyl)-(2-methoxycarbonyl-4-dimethylaminophenyl)methane-dissolved oil.

This microcapsule slurry was coated on one side of a normal paper by means of a wire bar coater to form a layer of uniform thickness, followed by drying to obtain a CB sheet.

The initial color density of the CB sheet thus obtained was measured by means of a Macbeth reflection color density tester RD-918 and found to be 0.06.

Further, this CB sheet was exposed to fade meter light for 10 minutes, or exposed at 40 ° C under a relative humidity of 90% for 24 hours, or exposed at 100 ° C for 10 hours. Then, the color densities of the respective CB sheets were measured and found to be 0.07, 0.06 and 0.06, respectively.

Further, a fresh CB sheet to examine the color forming performance and a CB sheet exposed to fade meter light for 10 minutes to examine the light resistance color forming performance, were respectively put on clay-type developer sheets as typical commercially available pressure sensitive developer sheets, followed by pressing under a pressure of 500 kg/cm$^2$ at 28 mmØ by a hydraulic pressing machine, whereby

developed blue color record images were obtained respectively.

The color densities of the record images thus obtained were measured and found to be 0.84 and 0.75, respectively.

Further, to examine the light resistance against color fading, a record image obtained by a fresh CB sheet was exposed to fade meter light for one hour, and then the reflection density of the record image was measured and found to be 0.81.

The results thus obtained are shown in Table 2 together with the results obtained in the above Comparative Example 2.

Table 2

| CB Sheet | | |
|---|---|---|
| | Example 2 | Comparative Example 2 |
| Initial color density of fresh CB sheet | 0.06 | 0.05 |
| Light resistance 10 min. | 0.07 | 0.24 |
| Humidity resistance 24 hr. | 0.06 | 0.06 |
| Heat resistance 10 hr. | 0.06 | 0.05 |

As is evident from Table 2, in Comparative Example 2, the initial color density of the fresh CB sheet was 0.05, whereas the color density after the light resistance test was as high as 0.24, and thus the initial color development of the CB sheet is substantial.

Whereas, in Example 2, as the color densities of from 0.06 to 0.07 after the tests for light resistance, humidity resistance and heat resistance are compared with the initial color density of 0.06 of the fresh CB sheet, there is no substantial change between before and after the respective tests, and thus the sheet had excellent properties without initial color development.

Further, in the same manner as the above test, a resin type developer sheet and a metal salt of salicylic acid derivative type developer sheet as typical commercially available pressure sensitive developer sheets, were tested for the light resistance against color fading.

The results are summarized in Tables 3 to 5.

Table 3

| Clay paper | | |
|---|---|---|
| | Example 2 | Comparative Example 2 |
| Color forming performance | 0.84 | 0.85 |
| Light resistance against color fading 1 hr. | 0.81 | 0.82 |
| Light resistance color forming performance 10 min. | 0.74 | 0.76 |

Table 4

| Resin paper | | |
|---|---|---|
| | Example 2 | Comparative Example 2 |
| Color forming performance | 0.73 | 0.56 |
| Light resistance against color fading 1 hr. | 0.67 | 0.55 |
| Light resistance color forming performance 10 min. | 0.63 | 0.53 |

Table 5

| Metal salt of salicylic acid sheet | | |
|---|---|---|
| | Example 2 | Comparative Example 2 |
| Color forming performance | 0.64 | 0.46 |
| Light resistance against color fading 1 hr. | 0.57 | 0.46 |
| Light resistance color forming performance 10 min. | 0.54 | 0.43 |

As is evident from Tables 3 to 5, with each of the commercially available pressure sensitive developer sheets, the developed color density was high, color fading by light was little, and the CB sheet had excellent properties without initial color development under exposure to light.

As is evident from Table 2 and Tables 3 to 5, the pressure sensitive recording sheet prepared by using the triarylmethane compound of Example 2 of the present invention was excellent in the solubility in oil, and the CB sheet was substantially free from initial color development. Further, the color fading of the record image under exposure to light was very little, and the CB sheet had excellent light resistance color forming performance. Thus, it was excellent in various properties such as the color forming performance and the storage stability.

Example 3

A CB sheet was prepared in the same manner as in Example 2 except that bis((4-dimethylamino)-phenyl)-(2-isopropoxycarbony-4-dimethylaminophenyl)methane was used instead of bis((4-dimethylamino)-phenyl)-(2-methoxycarbonyl-4-dimethylaminophenyl)methane.

Various properties of the CB sheet and the record image were examined in the same manner as in Example 2. The results are shown in Tables 6 to 9. As shown in these Tables, the CB sheet showed the same excellent properties as in Example 2.

Example 4

A CB sheet was prepared in the same manner as in Example 2 except that bis((4-dimethylamino)-phenyl)-(2-n-butoxycarbony-4-dimethylaminophenyl)methane was used instead of bis((4-dimethylamino)-phenyl)-(2-methoxycarbonyl-4-dimethylaminophenyl)methane.

Various properties of the CB sheet and the record image were examined in the same manner as in Example 2. The results are shown in Tables 6 to 9. As shown in these Tables, the CB sheet showed the same excellent properties as in Example 2.

Table 6

| CB Sheet | | |
|---|---|---|
| | Example 3 | Example 4 |
| Initial color density of fresh CB sheet | 0.06 | 0.06 |
| Light resistance 10 min. | 0.07 | 0.07 |
| Humidity resistance 24 hr. | 0.06 | 0.06 |
| Heat resistance 10 hr. | 0.06 | 0.06 |

Table 7

| Clay paper | | |
|---|---|---|
| | Example 3 | Example 4 |
| Color forming performance | 0.79 | 0.83 |
| Light resistance against color fading 1 hr. | 0.75 | 0.75 |
| Light resistance color forming performance 10 min. | 0.73 | 0.73 |

Table 8

| Resin paper | | |
|---|---|---|
| | Example 3 | Example 4 |
| Color forming performance | 0.61 | 0.68 |
| Light resistance against color fading 1 hr. | 0.55 | 0.60 |
| Light resistance color forming performance 10 min. | 0.54 | 0.61 |

Table 9

| Metal salt of salicylic acid sheet | | |
|---|---|---|
| | Example 3 | Example 4 |
| Color forming performance | 0.60 | 0.63 |
| Light resistance against color fading 1 hr. | 0.53 | 0.55 |
| Light resistance color forming performance 10 min. | 0.51 | 0.53 |

As described in the foregoing, the present invention provides a recording material which, when employed for a pressure sensitive recording sheet, presents a very clear blue color in a developed state and which is excellent in the solubility in capsule oil and excellent in the color forming performance and various properties such as the storage stability represented by the resistance against initial color development of the CB sheet.

Further, the present invention meets with the demand of a new market and thus has an effect of enlarging the application field of the recording material.

**Claims**

**1.** A triarylmethane compound of the following formula (I):

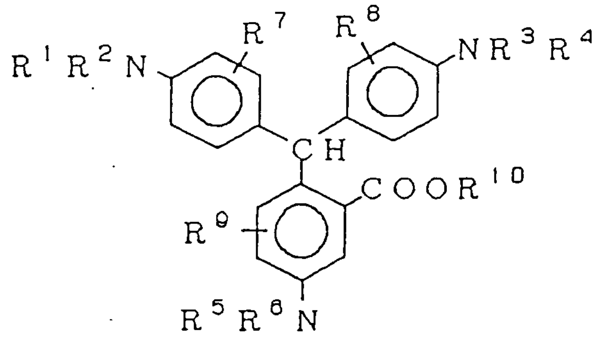

(I)

wherein each of $R^1$, $R^2$, $R^3$, $R^4$, $R^5$ and $R^6$ independently represents a $C_{1-8}$ alkyl group, a cycloalkyl group, an alkoxyalkyl group, or a benzyl or phenyl group which may be substituted by a halogen atom, an alkyl group or an alkoxy group, each of $R^7$, $R^8$ and $R^9$ independentlyrepresents a hydrogen atom, a halogen atom, an alkyl group, an alkoxy group, or an alkoxyalkyl group, and $R^{10}$ represents a $C_{1-8}$ alkyl group, an alkoxyalkyl group, a cycloalkyl group, or a benzyl or phenyl group which may be substituted by a halogen atom, an alkyl group, an alkoxy group or an alkoxyalkyl group.

**2.** The compound according to Claim 1, which is bis((4-dimethylamino)phenyl)-2-methoxycarbonyl-4-dimethylaminophenyl)methane.

**3.** The compound according to Claim 1, which is bis((4-dimethylamino)phenyl)-(2-isopropoxycarbonyl-4-dimethylaminophenyl)methane.

**4.** The compound according to Claim 1, which is bis((4-dimethylamino)phenyl)-(2-n-butoxycarbonyl-4-dimethylaminophenyl)methane.

**5.** The compound according to Claim 1, which is bis((2-methyl-4-dimethylamino)phenyl)-(2-methyoxycarbonyl-4-dimethylaminophenyl)methane.

**6.** The compound according to Claim 1, which is bis((2-methyl-4-dimethylamino)phenyl)-(2-ethyoxycarbonyl-4-dimethylaminophenyl)methane.

**7.** A pressure sensitive recording material comprising at least one member selected from the group consisting of triarylmethane compounds of the following formula (I)

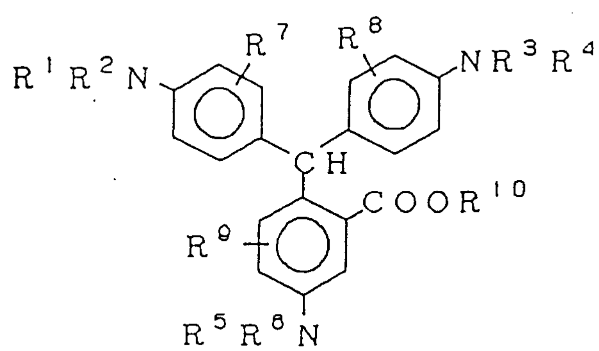

(I)

wherein each of $R^1$, $R^2$, $R^3$, $R^4$, $R^5$ and $R^6$ independently represents a $C_{1-8}$ alkyl group, a cycloalkyl group, an alkoxyalkyl group, or a benzyl or phenyl group which may be substituted by a halogen atom, an alkyl group or an alkoxy group, each of $R^7$, $R^8$ and $R^9$ independentlyrepresents a hydrogen atom, a halogen atom, an alkyl group, an alkoxy group, or an alkoxyalkyl group, and $R^{10}$represents a $C_{1-8}$ alkyl group, an alkoxyalkyl group, a cycloalkyl group, or a benzyl or phenyl group which may be substituted by a halogen atom, an alkyl group, an alkoxy group or an alkoxyalkyl group.

European Patent Office

# EUROPEAN SEARCH REPORT

Application Number

EP 91 10 5774

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PATENT ABSTRACTS OF JAPAN vol. 12, no. 52 (M-668)(2899) 17 February 1988, & JP-A-62 198494 (YAMADA KAGAKU KOGYO K.K.) 02 September 1987, * the whole document * --- | 1-7 | B41M5/136<br>C07C229/62 |
| A | CH-A-634259 (CIBA-GEIGY AG) * abstract * --- | 1, 7 | |
| A | PATENT ABSTRACTS OF JAPAN vol. 7, no. 269 (M-259)(1414) 30 November 1983, & JP-A-58 148789 (MITSUI TOATSU KAGAKU K.K.) 03 September 1983, * the whole document * --- | 1, 7 | |
| A | EP-A-58430 (MITSUI TOATSU CHEMICALS INC) * abstract * * page 9, line 22 - page 12, line 2 * ----- | 1 | |
| | | | TECHNICAL FIELDS SEARCHED (Int. Cl.5) |
| | | | B41M<br>C07C |

The present search report has been drawn up for all claims

| Place of search | Date of completion of the search | Examiner |
|---|---|---|
| THE HAGUE | 17 SEPTEMBER 1991 | MARKHAM R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document